# EUROPEAN PATENT SPECIFICATION

(11) **EP 1 031 315 B1**
(45) Date of publication and mention of the grant of the patent: **17.11.2004**
(21) Application number: 99301260.8
(22) Date of filing: 22.02.1999
(51) Int. Cl.: A61B 1/267

(54) **Laryngoscope**
Laryngoskop
Laryngoscope

(43) Date of publication of application: 30.08.2000
(73) Proprietor: Leoni, Carlo, 73469 Riesbürg (DE)
(72) Inventor: Cote, Nancy L., Gold River, CA 95670 (US)
(74) Representative: Papa, Elisabetta

(56) References cited:
- DE-U- 8 812 092
- FR-A- 414 137
- US-A- 3 005 452
- US-A- 3 771 514
- US-A- 4 611 579
- US-A- 5 873 818

## Description

### 1. Field of the Invention

The present invention relates generally to a laryngoscope for use by medical personnel for viewing the larynx, and, more particularly, to such a device having an expanded angular range of view.

### 2. Description of related Art

As to its major parts, a laryngoscope includes a handgrip and integrally related blade portion in an overall generally L-shape arrangement. Light directed along the blade portion to aid examination typically is powered by a battery pack contained within the handgrip. In use, the blade portion is inserted into a patient's mouth lifting the tongue and mandible and removing them from obstructing the view of the larynx.

In addition to enabling visual examination of the mouth and larynx, a primary advantageous use of this instrument is to facilitate insertion of an endotracheal tube into the trachea.

A continuing difficulty in use of laryngoscopes in the past has been to obtain a satisfactory view of the larynx considering the usual space restrictions to be expected, the rather difficult viewing angle at all times, and variation in anatomical features encountered among patients.

### SUMMARY OF THE INVENTION

It is, therefore, a primary aim and object of the present invention to provide a laryngoscope having an improved field of view.

In the practice of the present invention there are provided a laryngoscope with handgrip and blade portions and an optical system mounted on the blade portion providing a wide-angle field of view to one making an examination. The enlarged field of view is bent (angled) toward the outer end of the blade portion which would otherwise be out of view.

A further aspect of the invention is the provision of a light pipe mounted on the blade portion for directing a beam of light into the region of examination interest.

### BRIEF DESCRIPTION OF THE DRAWING

The ensuing description of a preferred embodiment of the invention can be more particularly understood by those skilled in the appertaining arts on making reference to the following description with further reference to the accompanying drawing in which:
FIG. 1 is a schematic depiction of a laryngoscope being used to view a patient's larynx;
FIG. 2 is a perspective view of a laryngoscope of the present invention;
FIG. 3 is a partially fragmentary, exploded view of the invention of FIG. 2;
FIG. 4 is an elevational view;
FIG. 5 is a further elevational view taken from the side opposite that of FIG. 4; and
FIG. 6 is a schematic representation of an optical system for use in the invention.

### DESCRIPTION OF A PREFERRED EMBODIMENT

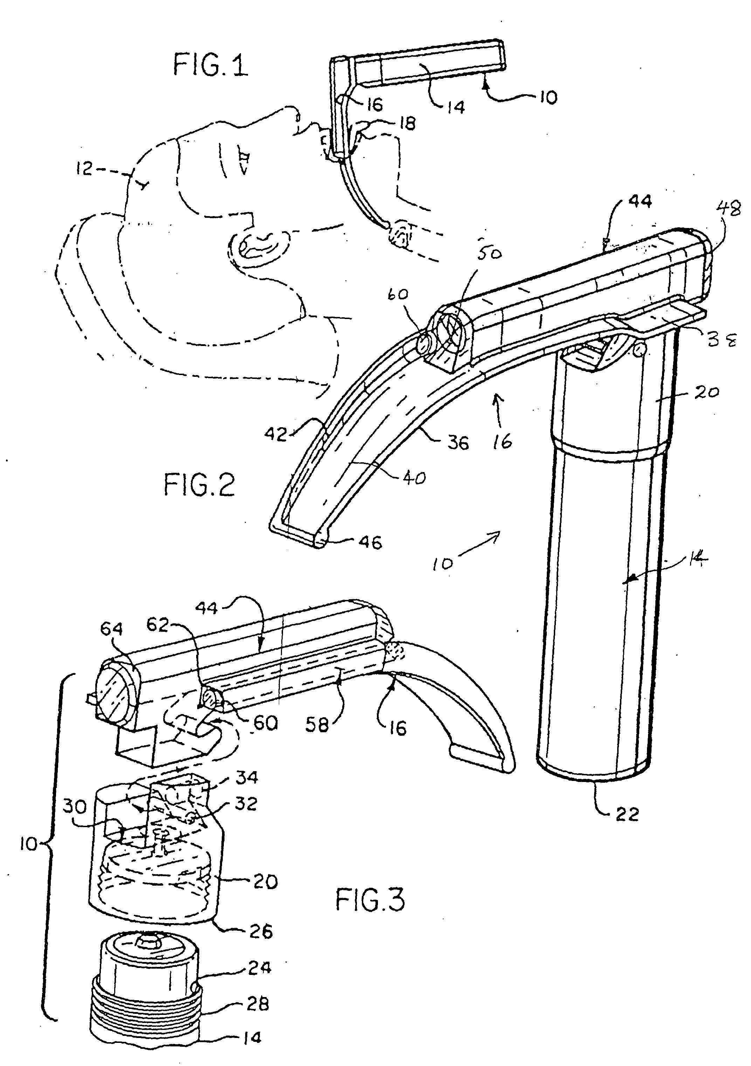

Turning now to the drawing and particularly FIG. 1, there is shown the laryngoscope instrument of the present invention enumerated as 10 placed in the mouth of a patient 12 for viewing the larynx 13 and to aid in the insertion of an endotracheal tube. The instrument includes a handle or handgrip 14 and a blade portion 16, the latter being used to lift the tongue and mandible 18. Also, on occasion the blade portion is levered on the front teeth of the patient in order to see the larynx.

With reference now particularly to FIGS. 2 and 3, the instrument 10 is seen to specifically include three separable parts, namely, the handle 14, blade portion 16 and a handle cap 20. As will be more specifically described, these different parts can be readily assembled together for use.

The handle 14 is essentially a hollow tube having a closed lower or outer end 22 and an open upper end 24. One or several dry cell batteries (not shown) are typically located within the handle cavity to provide power for an examination light to be described.

The cap 20 is a hollow cylindrical member with one end 26 open and containing internal threads for mating with threads 28 plus a washer or O-ring (not shown) to sealingly close off the handle open end 24. The upper or closed end of the cap has a parallel-sided slot 30 with a securement pin 32 having its ends affixed to the parallel sides and spaced from the slot bottom. A light source shown schematically at 34 is automatically interconnected with the battery power source in the handle upon full assembly of instrument. The securement technique between. the handle, cap and blade portion can be identical to that disclosed in U.S. patent 5,501,651. FLUID SUBMERSIBLE LARYNGOSCOPE PREVENTING ELECTROLYTIC CURRENT FLOW.

The blade portion 16 as seen in elevation (FIGS. 2-5) includes an elongated flat base 36 which has a substantially straight part 38 adjacent where it is connected to the cap 20 and an integral part 40 which curves generally toward the handle. A stiffening sidewall 42 extends along one lateral edge of the base. Overall blade surface features and shape for accomplishing tongue and mandible elevation during examination of a patient can be the same as disclosed in the 5,501,651 patent.

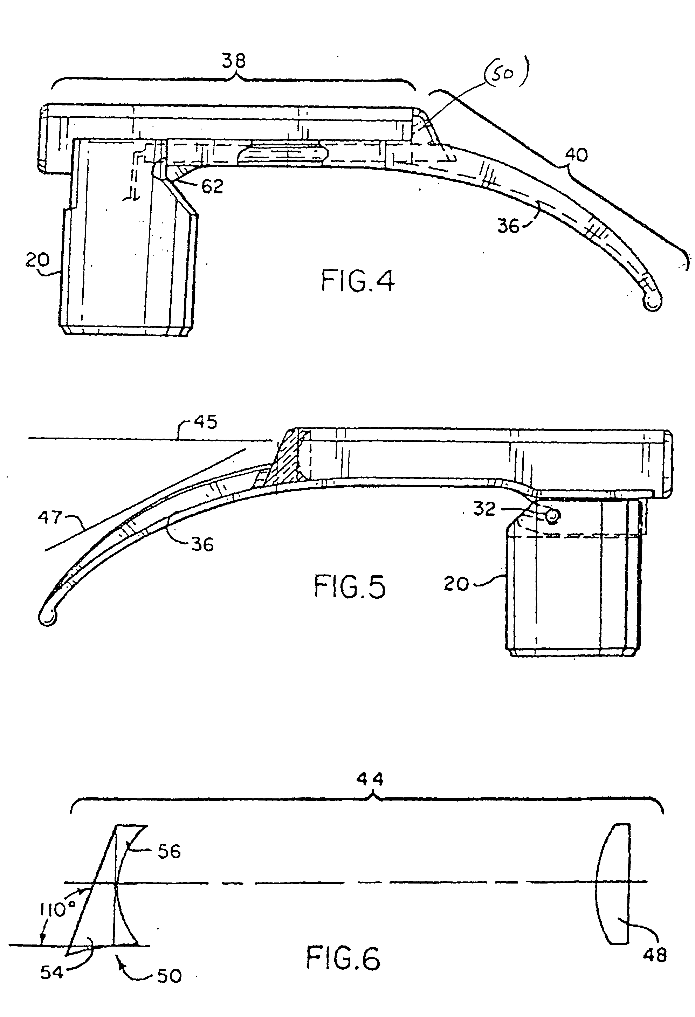

A primary contribution of this invention is the addition of an optical system 44 to the blade portion 16 which provides a wider angle of view to the one making an examination than is possible by taking an otherwise unassisted view along the blade. More particularly, the system 44 provides a view that extends beyond and just above the outer end 46 of the blade portion. As can be best seen in FIG. 5, the normal line of sight 45 (i.e., unassisted by optics) is considerably higher than desired for viewing the larynx. On the other hand, with the described optics the new line of sight 47 is directed downwardly substantially from the normal line 45, and therefore, requires at most a very minor physical shifting of the instrument to have a satisfactory view of the larynx.

For the ensuing detailed description of the optical system 44, reference is made to FIG. 6. The system includes a plano-convex eyepiece lens 48 which as the name implies is located at a point that can be conveniently accommodated to the eye of a user of the instrument preferably at a point just rearwardly of the handle 14. A prism-lens 50 serves as the forwardmost optic in the system located approximately at the point of demarcation between the flat base straight part 38 and curved part 40. The front face of the prism slopes rearwardly from bottom to top at a total angle of 110 degrees.

A hollow tube 52 affixed to the blade portion base 36 has the eyepiece lens48 received in one end and the prism-lens 50 in the other end. The tube 52 may be separately constructed and suitably secured to the base 36. Alternatively, the tube and base 36 may be constructed unitarily from metal or plastic (e.g., injection molding).

In a practical construction of an optical system 44 for the present invention, the eyepiece was constructed of optical quality acrylic having a smooth flat surface facing the user's eye and an opposite surface having a radius of 54.3337 mm providing a focal length of +110 mm. The prism-lens 50 was constructed of optical grade acrylic and consisted of a prism optic 54 cemented to a further lens 56 having a concave surface with a radius of 12.3555 mm facing the eyepiece. Prism optic 54 and lens 56 when assembled together (or optionally molded in one piece) provide a smooth sloping prism face of 110 degrees to the system axis, and has a focal length of -25 mm for the prism/lens combination. The prism and lenses may be optionally made of glass, or a number of transparent plastics, e.g., polycarbonate, acrylic, or crystalline polystyrene.

With respect to the functional operation of the optical system 44, the concave portion of lens 56 serves to produce a wide-angle view, while the companion prism lens 54 directs the view toward the blade tip 46 to better expose the larynx. Also, the lens 56 "miniaturizes" objects view, while the eyepiece lens 48 compensates for the miniaturization as well as providing for focusing.

A further aspect of the present invention is the provision of a light direction means 58 which extends from the light source 34 along a path generally parallel to the optical axis of the optical system 44 and closely spaced thereto (FIGS. 3 and 4). Specifically, the direction means includes a light pipe 60 consisting preferably of a cylindrical rod of a good light transmitting material (e.g., optical quality acrylic). The light pipe is located within a tubular housing 62 which can be secured to the optical system housing 64, or, alternatively, the two housings can be unitarily constructed with the blade base 36. The forward end 64 of the light pipe is preferably located forwardly of the prism 54 so as not produce any undesirable glare in the optical system. Moreover, the forward end face 64 is sanded or etched to produce diffused light which reduces glare or undesirable back reflection.

In accordance with the present invention there is provided laryngoscopic apparatus which overcomes certain prior existing deficiencies in such equipment, especially viewing difficulties encountered when the apparatus is being used in connection with insertion of an endotracheal tube into a trachea. The described apparatus achieves a highly expanded viewing region and bends or angles the line of sight toward the outer end of the blade; thereby approximating a direct line of sight view of the larynx and trachea.

Although the present invention is described herein in connection with a preferred embodiment, it is to be understood that those skilled in the appertaining arts may contemplate changes that come within the scope of the appended claims.

## Claims

1. Laryngoscope apparatus comprising a handle (14), a blade portion (16) and an optical system (44) mounted to the blade portion, the optical system including an eyepiece (48), a prism lens (54) spaced forwardly from the eyepiece (48) along a straightline optical axis providing visual examination of the trachea and adjacent tissue lying beyond the range of normal visibility of an observer viewing directly along the optical axis, the prism lens (54) having a surface facing forwardly in the direction of an object of interest, which surface is flat and canted at an angle different from 90 degrees to the optical axis; and a further lens (56) located between the prism lens (54) and eyepiece (48) with a concave lens surface facing said eyepiece (48).

2. Laryngoscope apparatus according to claim 1, further including a light source, a light pipe (60) having a first end in light receiving relation to said light source and a second end positioned to direct light received from the light source generally toward an outer end of the blade portion (16).

3. Laryngoscope apparatus according to claim 2, in which the second end of the light pipe (60) has a light diffusing surface.

4. Laryngoscope apparatus according to claim 2 or 3, in which the second end of the light pipe (60) is located forwardly of the prism lens (54) closer to the outer end of the blade portion (16).

5. Laryngoscope apparatus according to claim 2, 3 or 4, in which the light pipe (60) includes a hollow housing (62) with open ends and a rod constructed of a good light transmitting material.

6. Laryngoscope apparatus according to claim 5, in which the rod material is acrylic.

7. Laryngoscope apparatus, comprising:
a blade portion (16) having a generally straightline part (38) and a curved part (40) integral therewith;
a handle (14) affixed to the straightline part (38) of the blade portion (16) and including a light source (34);
housing means (64) affixed to the blade portion (16) having first and second elongated cavities extending along the straightline part (38) generally parallel to one another;
an eyepiece lens (48) including a plano-convex lens and a prism lens (54) arranged in the housing means first cavity in spaced apart relation, the prism lens having a forward facing flat surface canted at an angle of approximately 110 degrees to the straightline part of the blade portion, a back surface and a further lens (56) adhered to the prism back surface having a concave surface facing the eyepiece lens; and
a light pipe (60) received in the second cavity having a first end in light receiving relation to the light source (34) and a second end located outwardly beyond the prism lens (54).

8. Laryngoscope apparatus according to claim 7 in which the light pipe (60) includes a length of a good light transmitting material the second end of which has a light diffusing surface.

9. Laryngoscope apparatus according to claims 7 or 8 in which the light pipe second end includes a flat end surface canted with respect to the light pipe axis.

## Patentansprüche

1. Laryngoskopvorrichtung, die einen Griff (14), einen Spatelabschnitt (16) und ein optisches System (44) umfasst, das an dem Spatelabschnitt angebracht ist, wobei das optische System ein Okular (48), eine Prismenlinse (54), die auf einer geradlinigen optischen Achse vor dem Okular (48) beabstandet angeordnet ist und visuelle Untersuchung der Luftröhre und von angrenzendem Gewebe ermöglicht, das jenseits des Bereiches normaler Sichtbarkeit eines Betrachters liegt, der direkt entlang der optischen Achse blickt, wobei die Prismenlinse (54) eine Fläche hat, die in der Richtung des interessierenden Objektes nach vorn gewandt ist, und die Fläche plan und in einem von 90° verschiedenen Winkel zur optischen Achse geneigt ist, sowie eine weitere Linse (56) enthält, die sich zwischen der Prismenlinse (54) und dem Okular (48) befindet, wobei eine konkave Linsenfläche dem Okular (48) zugewandt ist.

2. Laryngoskopvorrichtung nach Anspruch 1, die des Weiteren eine Lichtquelle, einen Lichtleiter (60) enthält, der ein erstes Ende, das in Lichtempfangsbeziehung zu der Lichtquelle ist, und ein zweites Ende aufweist, das so angeordnet ist, dass es von der Lichtquelle empfangenes Licht allgemein auf ein äußeres Ende des Spatelabschnitts (16) zu leitet.

3. Laryngoskopvorrichtung nach Anspruch 2, wobei das zweite Ende des Lichtleiters (60) eine lichtstreuende Fläche aufweist.

4. Laryngoskopvorrichtung nach Anspruch 2 oder 3, wobei sich das zweite Ende des Lichtleiters (60) vor der Prismenlinse (54) näher an dem äußeren Ende des Spatelabschnitts (16) befindet.

5. Laryngoskopvorrichtung nach Anspruch 2, 3 oder 4, wobei der Lichtleiter (60) ein hohles Gehäuse (62) mit offenen Enden und einen Stab enthält, der aus gut lichtdurchlässigem Material aufgebaut ist.

6. Laryngoskopvorrichtung nach Anspruch 5, wobei das Stabmaterial Acryl ist.

7. Laryngoskopvorrichtung, die umfasst:
einen Spatelabschnitt (16) mit einem im Allgemeinen geradlinigen Teil (38) und einem gekrümmten Teil (40), der integral damit ausgebildet ist;
einen Griff (14), der an dem geradlinigen Teil (38) des Spatelabschnitts (16) befestigt ist und eine Lichtquelle (34) enthält;
eine Gehäuseeinrichtung (64), die an dem Spatelabschnitt (16) befestigt ist und einen ersten sowie einen zweiten länglichen Hohlraum aufweist, die sich entlang des geradlinigen Teils (38) im Allgemeinen parallel zueinander erstrecken;
eine Okularlinse (48), die eine plankonvexe Linse und eine Prismenlinse (54) enthält, die in dem ersten Hohlraum der Gehäuseeinrichtung voneinander beabstandet angeordnet sind, wobei die Prismenlinse eine nach vorn gerichtete plane Fläche, die in einem Winkel von ungefähr 110 Grad zu dem geradlinigen Teil des Spatelabschnitts geneigt ist, eine hintere Fläche, sowie eine weitere Linse (56) aufweist, die an der hinteren Fläche des Prismas angebracht ist und eine konkave Fläche hat, die der Okularlinse zugewandt ist; und
einen Lichtleiter (60), der in dem zweiten Hohlraum aufgenommen ist und ein erstes Ende in Lichtempfangsbeziehung zu der Lichtquelle (34) sowie ein zweites Ende aufweist, das sich außerhalb jenseits der Prismenlinse (54) befindet.

8. Laryngoskopvorrichtung nach Anspruch 7, wobei der Lichtleiter (60) einen Abschnitt aus gut lichtdurchlässigem Material enthält, dessen zweites Ende eine lichtstreuende Fläche aufweist.

9. Laryngoskopvorrichtung nach Anspruch 7 oder 8, wobei das zweite Ende des Lichtleiters eine plane Abschlussfläche enthält, die in Bezug auf die Lichtleiterachse geneigt ist.

## Revendications

1. Laryngoscope comprenant une poignée (14), une partie de lame (16) et un système optique (44) monté sur la partie de lame, le système optique comprenant un oculaire (48), une lentille à prisme (54) espacée vers l'avant à partir de l'oculaire (48) le long d'un axe optique linéaire permettant un examen visuel de la trachée et des tissus adjacents se trouvant au-delà de la portée de visibilité normale d'un observateur visualisant directement le long de l'axe optique, la lentille à prisme (54) ayant une surface tournée vers l'avant en direction d'un objet d'intérêt, dont la surface est plate et oblique à un angle différent de 90 degrés par rapport à l'axe optique ; et une autre lentille (56) située entre la lentille à prisme (54) et l'oculaire (48) avec une surface de lentille concave faisant face audit oculaire (48).

2. Laryngoscope selon la revendication 1, comprenant en outre une source de lumière, un conduit de lumière (60) ayant une première extrémité dans un rapport de réception de lumière par rapport à ladite source de lumière et une seconde extrémité positionnée de façon à diriger la lumière reçue de la source de lumière généralement vers une extrémité externe de la partie de lame (16).

3. Laryngoscope selon la revendication 2, dans lequel la seconde extrémité du conduit de lumière (60) a une surface qui renvoie la lumière.

4. Laryngoscope selon la revendication 2 ou 3, dans lequel la seconde extrémité du conduit de lumière (60) est située vers l'avant de la lentille à prisme (54) plus près de l'extrémité externe de la partie de lame (16).

5. Laryngoscope selon la revendication 2, 3 ou 4, dans lequel le conduit de lumière (60) comprend un boîtier creux (62) avec des extrémités ouvertes et une tige construite à partir d'un matériau bon conducteur de lumière.

6. Laryngoscope selon la revendication 5, dans lequel le matériau de la tige est de l'acrylique.

7. Laryngoscope, comprenant :
une partie de lame (16) ayant une partie généralement linéaire (38) et une partie courbée (40) intégrées à celle-ci ;
une poignée (14) fixée à la partie linéaire (38) de la partie de lame (16) et comprenant une source de lumière (34) ;
un boîtier (64) fixé à la partie de lame (16) ayant des première et seconde cavités allongées se prolongeant le long de la partie linéaire (38) généralement parallèle l'une à l'autre ;
une lentille de l'oculaire (48) comprenant une lentille plano-convexe et une lentille à prisme (54) disposées dans la première cavité du boîtier dans un rapport espacé, la lentille à prisme ayant une surface plate tournée vers l'avant oblique à un angle de 110 degrés ou approximativement par rapport à la partie linéaire de la partie de lame, une surface arrière et une autre lentille (56) collées à la surface arrière à prisme ayant une surface concave tournée vers la lentille de l'oculaire ; et
un conduit de lumière (60) reçu dans la seconde cavité ayant une première extrémité dans un rapport de réception de lumière par rapport à la source de lumière (34) et une seconde extrémité située vers l'extérieur au-delà de la lentille à prisme (54).

8. Laryngoscope selon la revendication 7 dans lequel le conduit de lumière (60) comprend une longueur d'un matériau bon conducteur de lumière dont la seconde extrémité a une surface qui renvoie la lumière.

9. Laryngoscope selon les revendications 7 ou 8 dans lequel la seconde extrémité du conduit de lumière comprend une surface d'extrémité plate oblique par rapport à l'axe du conduit de lumière.
